# EUROPEAN PATENT SPECIFICATION

(11) **EP 0 327 263 B1**
(45) Date of publication and mention of the grant of the patent: **07.09.1994**
(21) Application number: 89300785.6
(22) Date of filing: 27.01.1989
(51) Int. Cl.: A61K 7/06, A61K 47/00

(54) **Hair growth stimulation with nitroxide and other radicals**
Haarwachstumanregung mit Nitroxyd und anderen Radikalen
Stimulation de la croissance des cheveux avec nitroxide et autres radicaux

(30) Priority: 29.01.1988 US 149720
(43) Date of publication of application: 09.08.1989
(73) Proprietor: PROCTOR, Peter H., Suite 1616 Houston, TX 77027 (US)
(72) Inventor: PROCTOR, Peter H., Suite 1616 Houston, TX 77027 (US)
(74) Representative: Gore, Peter Manson

(56) References cited:
- EP-A- 0 273 202
- WO-A-83/02558
- WO-A-86/00616
- WO-A-87/00427
- GB-A- 2 198 132
- US-A- 3 551 554

## Description

This invention relates to a composition and method for stimulating hair growth, and to a kit for preparation of such a composition.

Various treatments have been available for stimulating the cosmetic growth of hair, and for conditions such as male and female pattern baldness and alopecia areata. Several substances were known to be effective when administered internally, but had undesirable concomitant systemic effects and the hypertrichosis was not confined to the scalp area. In an effort to avoid these side effects and to confine the hypertrichosis to the scalp area, several attempts were made to apply such substances in a topical preparation to the affected area. However, such attempts had generally been only marginally successful, and the results obtained with the topical preparation containing the orally effective substances were comparable to and generally little better than these obtained with topical application of the carrier only.

WO87/00427 describes a topical composition and method for stimulating hair growth. The composition contains, in an occlusive or semi-occlusive pharmaceutical carrier, miroxidil and a hydroxyl radical scavenger such as DMSO. The method involves applying the composition to the skin once a day.

WO/83/02558 describes the use of retisoids and minoxidil to increase the rate of hair growth. This is said to prolong the anagen phase of the hair cycle and to be suitable for treating certain types of alopoecia.

US-A-3551554 describes a method of enhancing tissue penetration of physiologically active agents, including physically active steroids, anti-neoplastic agents, antigens, anti-unicellular microorganism agents, antihistaminic agents, neuropharmacological agents, anti-inflammatory agents, anti-coagulants, vasodilators, u.v.-screening agents, diagnostic dyes and radiopaque agents and nutrients by conjointly applying them to the tissue with DMSO.

U.S. Patent 2,986,573 described a process for treating hypertension by administering a 1,2,4-benzothiadiazine 1,1-dioxide, otherwise unsubstituted in the heterocyclic portion of the nucleus, having a saturated lower aliphatic hydrocarbon radical in the 3-position and a chlorine atom or its equivalent on the benzenoid portion of the nucleus in the 6- or 7-position.

U.S. Patent 4,184,039 described the development of uncontrolled hair growth in patients treated orally with 1,2,4-benzothiadiazine 1,1-dioxides; and also described topical application of 6-chloro-3-dimethylaminoethoxymethyl-2H-1,2,4-benzothiadiazine 1,1-dioxide and 6-chloro-3-cyclohexenyl-3,4-dihydro-2H-1,2,4-benzothiadiazine 1,1-dioxide in DMSO and in suspension to promote hair growth.

U.S. Patents 4,139,619 and 4,596,812 described a process for stimulating the growth of mammalian hair by the application of 6-amino-4-(substituted amino)-1,2-dihydro-1-hydroxy-2-iminopyrimidines to mammalian skin in association with a topical pharmaceutical carrier.

U.S. Patent 4,347,245 described a composition containing spironolactone in a liquid carrier such as alcohol, urea, mineral oil or white petrolatum.

Stewart, M.E. et al., "Antiandrogens and the Skin," International Journal of Dermatology, Vol. 17, pp. 167-179 (1978) described the application to the foreheads of acne patients of 10% cyproterone in 50% aqueous dimethyl sulfoxide, with no reduction in sebum secretion or improvement in acne being produced.

U.S. Patent 4,367,227 described a composition for reducing sebum secretion when applied to the skin, which composition contained cyproterone acetate dissolved in a C₂-C₃ aliphatic alcohol.

The present invention relates to hair growth stimulation, for example, in individuals with pattern hair loss. It has been discovered that certain free radical species can stimulate hair growth particularly when repeatedly applied in a topical preparation to the site at which stimulated hair growth is desired.

According to the present invention there is provided a method of stimulating hair growth by cosmetic treatments, comprising:
topically applying to skin a pharmacologically acceptable nitroxide radical-forming compound selected from nitroxide spin labels and spin traps and N-oxides of niacins; and
concurrently therewith, topically applying to the skin an adjuvant selected from reducing agents, antioxidants and hydroxyl radical scavengers.

Adjuvants include, for example, hydroxyl radical scavengers, reducing agents and antioxidants which may either induce formation of free radicals from the free radical-forming compounds, or stabilize the free radicals formed therefrom by limiting reaction with other reactive radicals. The topical application can also include a combination of such adjuvants, such as, for example, a reducing agent and superoxide dismutase activity, optimally, the adjuvant may also include an antiandrogen.

The present invention also provides a method of stimulating hair growth by cosmetic treatment, comprising:
mixing, in a pharmaceutical carrier (i) a pharmacologically acceptable stable nitroxide radical-forming compound selected from nitroxide spin labels and spin traps and N-oxides of niacins with (ii) an amount of pharmacologically acceptable reducing agent effective to induce nitroxide radical formation from the stable compound,
applying the nitroxide-forming mixture topically to skin, and
repeating application over a period of time sufficient to stimulate hair growth from the skin.

In another aspect, the invention provides a kit for preparing a unit dosage of a hair growth stimulating preparation, comprising:-
a unit dosage amount of a pharmacologically acceptable nitroxide radical forming compound selected from organic nitrates and inorganic nitrates in a pharmaceutical carrier, the reducing agent being reactive with the radical-forming compound to form nitroxide radicals, means for separating the dosage amounts from each other prior to dispensing, and means for dispensing the unit dosage amounts. The dispensing means may also include means for mixing the unit dosage amounts. The kit can be used to prepare a topical preparation which has been activated to generate nitric oxide radicals at the site of application to stimulate hair growth following repeated applications.

In still another aspect, the invention provides a composition for topical stimulation of hair growth from skin which includes in association with a topical pharmaceutical carrier, a pharmacologically acceptable stable radical-forming compound selected from nitroxide spin labels and N-oxides of niacins and a pharmacologically acceptable adjuvant. The adjuvant comprises a hydroxyl radical scavenger, a reducing agent, an antioxidant and/or an antiandrogen.

The method of the present invention involves the concurrent topical application of a stable free radical-forming compound as a hair growth stimulant and adjuvant which either activates the free radical formation and/or protects the stable free radicals once formed from reaction with other reactive species such as hydroxyl radicals, for example. The hair growth stimulant and adjuvant are usually repeatedly applied to the scalp or other site at which hair growth is desired. The application is typically made once or twice daily, although a greater or lesser frequency of application can be used, if desired. Generally, the frequency of application is greater during the first three to six or twelve months of treatment, e.g. twice a day, but the application frequency usually can be reduced to once every two to three days as a maintenance program after the desired hair growth stimulation has been achieved.

The carrier or carriers in which the free radical-forming compound, adjuvant and any other active ingredients will generally be substantially homogenously dispersed, e.g. at 10-100 mM, is preferably an occlusive or semi-occlusive preparation which may be a water-in-oil emulsion, but is most preferably an oil-in-water emulsion. As used herein, the terms "occlusive" or "semi-occlusive" are used in reference to a carrier which substantially prevents or inhibits, respectively, evaporation of water from the skin to which it is applied. As examples of non-occlusive carriers, there may be mentioned water, urea, alcohols and glycols such as methanol, ethanol, propanol, butanol, ethylene glycol and propylene glycol, and the like. Suitable water-in-oil emulsions are commercially available under the designations Aquaphor*, cold cream, Eucerin,* hydrous lanolin, Hydrosorb,* hydrophilic petrolatum, Nivea*, Polysorb*, Qualatum* and Velvachol*. Suitable oil-in-water emulsions are available commercially under the designations acid mantle cream, Almay* emulsion cream, Cetaphil*, Dermabase*, Dermovan*, hydrophilic ointment, Keri* cream, Lubriderm* cream, Multibase* cream, Neobase* cream, Univase* cream, Vanibase* cream, and Wibi*. The carrier may further contain various other emollients, emulsifiers, water, perfumes, colorants, preservatives and the like. In a preferred embodiment, the carrier comprises the Dermovan* emulsion, propylene glycol and water.
(* when used herein indicates Trade Mark)

According to the method of the invention, the free radical source and adjuvant are concurrently applied topically to the skin to be treated, such as the scalp. Preferably, the application is once a day with a sufficient amount of the free radical source and adjuvant to cover the area at which the stimulation of hair growth is desired. Generally, results are improved when the active ingredients are applied after water-soaking the skin. Thus, a preferred embodiment of the method is convenient in that the free radical source and adjuvant can be applied once daily immediately following bathing. However, it is desirable to leave the medication in place for a period of time and it should not be washed off for at least several hours, probably at least eight hours. Generally, best results are obtained in treatment of balding or thinly-haired scalp areas in which hair loss has not occurred for a period of time substantially in excess of about three to five years. The effectiveness also depends, although to a lesser degree, inversely on the age of the user. As used herein, the term "hair growth stimulation" generally includes conversion of vellus hairs to terminal hairs, an increase in shaft diameter, an increase in the rate of growth of terminal and vellus hairs, and possibly follicular neogenesis.

In one embodiment of the present invention, sodium nitrite is applied to the scalp, followed by a separate application of a reducing agent such as cysteine.

By concurrent application, it is meant that the free radical source and adjuvant are applied together or at or near the same time so that the adjuvant functions with the free radical source to enhance the hair growth stimulation relative to that which would be obtained by application of the free radical source or the adjuvant alone. Generally, the adjuvant will be active in mixture, or in the skin to which it is applied, with the free radical source to form free radicals by reaction therewith, or otherwise by shifting equilibrium between the excited state or free radical form of the free radical source toward a greater preponderance of the excited state species. Alternatively, the adjuvant may be active in the presence of the free radicals formed from the free radical source compound to protect the same by reacting with other undesired free radicals, or inhibiting formation thereof, which undesired free radicals might otherwise react with or inactivate the desired free radicals. In this manner the free radicals formed from the free radical source are available in the skin to stimulate hair growth. For example, the free radical source compound and adjuvant may be premixed in the same carrier, mixed at or near the time of their application to the skin, or applied separately within a period of time sufficient for concurrent activity, e.g. within several minutes to one hour or more of each other.

Free radicals are molecules having one or more electrons with unpaired spins and are generally short-lived, reactive or unstable chemical species, conventionally having a half life of the order of less than about 10 msec. However, relatively stable free radicals having a half life much longer than this can be formed in some species due to steric protection, resonance stabilization and other means of protecting the unpaired electron. For example, nitric oxide radicals have a half-life of the order of 30 seconds in biological systems, while spin labels and traps are, or may form, free radicals which are nearly indefinitely stable. The formation of stable free radicals in a substance (other than a spin label) is generally attributable to electron acceptance or donation from other radicals or reducing and/or oxidizing species, and is generally confirmed by electron spin resonance spectrometry.

Stable free radical-forming compounds generally include resonance stabilized nitroxy compounds, sulfoxy compounds, other nitrogenous compounds and the like. A preferred class of compounds include the nitroxide radical-forming compounds such as nitrovasodilators and spin traps and labels. As exemplary nitrovasodialtors which may form relatively stable nitroxide radical species there may be mentioned, nitroglycerin, amyl nitrate, sodium nitroprusside, sodium nitrite, and the like.

Other stable nitroxide radical-forming compounds preferred as hair growth stimulants in the present invention include nitroxide spin labels and spin traps. Exemplary of these are: melanin; 4,4-dimethyl-3-oxazolinyloxy (hereinafter "doxyl") and derivatives such as 3-doxyl-5α-cholestane, 3-doxyl-17β-hydroxy-5α-androstane, 5-doxylstearic acid, 7-doxylstearic acid, 12-doxylstearic acid, 16-doxylstearic acid, 5-doxylstearic acid methyl ester, 7-doxylstearic acid methyl ester, 12-doxylstearic acid methyl ester, 16-doxylstearic acid methyl ester and the like; 2,2,5,5-tetramethyl-1-pyrrolidinyloxyl (hereinafter "proxyl") and derivatives such as 3-(aminomethyl)-proxyl, 3-(2-[2-bromoacetamido]-acetamido)-proxyl, 3-(2-[2-2-bromoacetamido)-ethoxyethyl]-carbamoyl)-proxyl, 3-(2-bromoacetamido]-methyl)-proxyl, 3-(3-[2-bromoacetamido]-propylcarbamoyl)-proxyl, 3-(2-bromoacetamido)-proxyl, 3-carbamoyl-proxyl, 3-carboxy-proxyl, 3-cyano-proxyl, 3-(5-[dimethylamino]-1-naphthalene-sulfonamido)-proxyl, 3-(5-fluoro-2,4-dinitroanilino)-proxyl, 3-(2-[2-iodoacetamido]-acetamido)-proxyl, 3-(2-[2-(2-iodoacetamido)-ethoxyethyl]-carbamoyl)-proxyl, 3-(2-iodoacetamidomethyl)-proxyl, 3-(3-[2-iodoacetamido]-propylcarbamoyl)-proxyl, 3-(2-iodoacetamido)-proxyl, 3-(2-[2-isothiocyanatoethoxy]-ethylcarbamoyl)-proxyl, 3-(2-isothiocyanatoethylcarbamoyl)-proxyl, 3-(isothiocyanatomethyl)-proxyl, 3-(3-isothiocyanato-propyl carbamoyl)-proxyl, 3-(2-[2-maleimidoethoxy]-ethylcarbamoyl)-proxyl, 3-(2-maleimidoethyl-carbamoyl)-proxyl, 3-(maleimidomethyl)-proxyl, 3-(3-maleimidopropyl-carbamoyl)-proxyl, 3-maleimidoproxyl, 3-(4-nitrophenoxy carbonyl)-proxyl, N,N'-bis(3-proxyl carbonyl)-1,2-ethanediamine, and the like; 2,2,6,6,-tetramethyl-1-piperidinyloxyl (hereinafter "tempo") and derivatives such as 4-amino-tempo, 4-(2-bromoacetamido)-tempo, 4-[N-formyl-N-(3-hydroxypropyl)amino]-tempo, 4-[N,N-bis(2-hydroxyethyl)]amino-tempo, 4-(ethoxyfluorophosphinyloxy)-tempo, 4-hydroxy-tempo, 4-(2-iodoacetamido)-tempo, 4-isothiocyanato-tempo, 4-maleimido-tempo, 4-(4-nitrobenzoyloxy)-tempo, 4-oxo-tempo, 4-phosphonooxy-tempo, N,N'-bis(4-tempo)oxamide, 1,2-ethanediaminetetraacetic acid 2',4'-bis[(CN-4-tempo)amide], N,N'-bis(4-tempo)-1,2-ethanediamine, N,N'-bis(4-tempo)decanediamide, and the like; other spin labels such as 2-(acetoxymercuri)-4,4,5,5-tetramethyl-2-imidazolin-1-yloxy-3-oxide, 3-carbamoyl-2,5-dihydro-2,2,5,5-tetramethyl-1H-pyrrol-1-yloxy, 3,([ethoxycarbonyl]-oxycarbonyl)-2,5-dihydro-2,2,5,5-tetramethyl-1H-pyrrol-1-yloxy and the like; and nitrone and nitroso spin traps such as N-t-butyl-α-phenyl-nitrone, 3,5-dibromo-4-nitroso-benzenesulfonic acid, 5,5-dimethyl -1-pyrroline N-oxide, 2-methyl-2-nitroso-propane, nitrosobenzene, nitrosodisulfonic acid, α-(4-pyridyl-1-oxide)-N-t-butylnitrone, 3,3,5,5-tetramethyl-pyrroline N-oxide, 2,4,6-tri-t-butylnitrosobenzene, and the like;
Such spin labels and spin traps are commercially available.

Other exemplary stable nitroxide radical-forming compounds include sodium azide, hydroxylamine, and the like. Particularly preferred nitroxide radical-forming compounds include, for example, nicotinamide N-oxide and nicotinic acid N-oxide, and the like.

Radical generating species reactive with reducing agents to form stable free radicals such as nitric oxide generally include hydroxylamine, organic nitrates, inorganic nitrites, peroxides, and the like. The organic nitrates and inorganic nitrites are preferred source radical compounds. Exemplary organic nitrates include trinitroglycerin, amyl nitrate, erithrithyl tetranitrate, isosorbide dinitrate, isosorbide-2-nitrate, isosorbide-5-nitrate, isomannide dinitrate, isomannide 2-nitrate, isoidide dinitrate, isoidide-2-nitrate, sodium nitruprosside and the like. Inorganic nitrites generally include alkali and alkaline earth metal nitrates and transition metal nitrates, such as, for example, sodium nitrite, potassium nitrite, lithium nitrite, and the like. Contemplated peroxides include benzoyl peroxide, hydrogen peroxide, urea hydrogen peroxide, carbamoyl peroxide, and the like.

The adjuvant is selected from hydroxyl radical scavengers, reducing agents, antioxidants and antiandrogens. The adjuvant used in the present method serves to induce formation of the free radical species of, or from, the foregoing compounds, to stabilize the free radicals once formed, and/or to protect the desired free radicals from reacting with other free radical species present in the skin, e.g. by scavenging or inhibiting formation of hydroxyl or other radicals with which the radical-forming compounds, or the radical formed therefrom, is reactive. Regardless of the mechanism involved, the adjuvant synergizes with the free radical-forming compound to enhance the hair growth stimulation thereof on repeated topical applications.

The type of adjuvant employed will depend on the particular type of free radical-forming compound employed. One class of adjuvants preferred for use in conjunction with the stable free-radical forming compounds described above is the group of compounds including radical scavengers, and particularly the pharmacologically suitable reducing agents and antioxidants which are generally effective hydroxyl radical scavengers.

As used herein, the term "free radical scavenger" includes compounds which suppress free radical generation as well as compounds which react with free radicals in biological systems. Hydroxyl radicals scavengers are, for example, sulfoxides, phosphine oxides, retinoids, purines, pyrimidines, thiols, halide ions, aromatic hydrocarbons and the like. Free radical scavengers suitable in the present invention include those pharmacologically acceptable hydroxyl radical scavengers which have a substantial effectiveness as a hydroxyl radical scavenger, preferably compounds having an effectiveness as a hydroxyl radical scavenger substantially equivalent to or better than DMSO, i.e. a specific reaction rate constant with hydroxyl radical on the order of 5-6 x 10⁹ dm³/mol-sec or higher, and particularly hydroxyl radical scavengers having a higher reactivity with hydroxyl radicals than nitric oxide, i.e. on the order of 8.9-11x10¹⁰ dm³/mol-sec or more.

One preferred class of reducing agents and free radical scavengers includes sulfoxides of the formula R⁸R⁹SO wherein R⁸ is alkyl, alkenyl, heteroalkyl (e.g. thiaalkyl or azaalkyl), hydroxyalkyl, or alkoxyalkyl having up to about 14 carbon atoms, and R⁹ is independently alkyl or hydroxyalkyl having from 1 to about 8 carbon atoms. Examples of R⁸ suitable herein include octyl, nonyl, decyl, undecyl, dodecyl, 3-decenyl, 2-dodecenyl, 3-undecenyl, 3-octenyl, 2-ketooctyl, 2-ketodecyl, 2-ketoundecyl, 2-ketododecyl, 2-hydroxyoctyl, 2-hydroxydecyl, 2-hydroxyundecyl, 2-hydroxydodecyl, 3-hydroxyundecyl, 3-methoxyundecyl, 2-methoxydodecyl, 3,6-dioxadodecyl, 2-ethylhexyl, and branched chain nonyl and dodecyl resulting from polymerization of three and four moles of propylene, respectively, and the like. Examples of R⁹ include methyl, ethyl, propyl, butyl, hydroxymethyl, 2-hydroxyethyl, 3-hydroxypropyl, and 4-hydroxybutyl, and the like.

Especially preferred sulfoxides for the purposes of this invention are the dialkyl sulfoxides where R⁸ is a hydrocarbyl alkyl or hydroxy-substituted alkyl group containing from 8 to 12 carbon atoms and R⁹ is methyl, ethyl or propyl. As examples of these preferred sulfoxides there may be mentioned octyl methyl sulfoxide, nonyl methyl sulfoxide, decyl methyl sulfoxide, undecyl methyl sulfoxide, dodecyl methyl sulfoxide, 2-hydroxydecyl methyl sulfoxide, 2-hydroxyundecyl methyl sulfoxide and 2-hydroxydodecyl methyl sulfoxide.

Another preferred class of reducing agents and hydroxyl radical scavengers includes the tertiary phosphine oxides of the formula R¹⁰R¹¹R¹²PO wherein R¹⁰ is alkyl, aralkyl heteroalkyl (e.g. azaalkyl or thiaalkyl), hydroxyalkyl, alkoxyalkyl, or ketoalkyl of from 1 to 14 carbon atoms, or aryl of from 6 to 12 carbon atoms, and R¹¹ and R¹² are independently alkyl, hydroxyalkyl, alkoxyalkyl or ketoalkyl of from 1 to 4 carbon atoms. Examples of R¹⁰ include methyl, ethyl, propyl, butyl, hexyl, heptyl, octyl, nonyl, decyl, undecyl, dodecyl, 2-propenyl, 3-decenyl, 2-dodecenyl, 3-undecenyl, 3-octenyl, 2-ketobutyl, 2-ketohexyl, 2-ketooctyl, 2-ketodecyl, 2-ketoundecyl, 2-ketododecyl, 2-hydroxypropyl, 2-hydroxyhexyl, 3-hydroxyheptyl, 2-hydroxyoctyl, 2-hydroxyundecyl, 2-hydroxydodecyl, 3-hydroxyundecyl, 2-methoxybutyl, 3-methoxyundecyl, 2-methoxydodecyl, 2-chlorodecyl, 3-chlorobutyl, 2-thiomethylhexyl, 3,6-dioxadodecyl, 2-oxaheptyl, 3-azahexyl, 2-thiadecyl, 2-ethylhexyl, phenyl, naphthyl, m-tolyl, benzyl, and branched chain nonyl and dodecyl resulting from polymerization of three and four moles of propylene, respectively.

Examples of R¹¹ and R¹² include methyl, ethyl, propyl, hydroxymethyl, 1-hydroxypropyl, 2-hydroxyethyl, and the like.

Especially preferred phosphine oxides for the purpose of this invention are those in which R¹⁰ is a hydrocarbyl alkyl or hydroxy-substituted alkyl substituent containing from 8 to 12 carbon atoms and R¹¹ and R¹² are each methyl, ethyl or propyl. As examples of these preferred phospine oxides there may be mentioned octyl dimethyl phosphine oxide, nonyl diethyl phosphine oxide, decyl dimethyl phosphine oxide, undecyl dimethyl phosphine oxide, dodecyl dimethyl phosphine oxide, 2-hydroxydecyl dimethyl phosphine oxide, 2-hydroxyundecyl dimethyl phosphine oxide and 2-hydroxydodecyl dimethyl phosphine oxide. Dodecyl dimethyl phosphine oxide is especially preferred.

The retinoids comprise another preferred class of free radical scavengers and reducing agents. Exemplary retinoids include carotene, tretinoin, isotretinoin, 9-cis-tretinoin, retinol, retinol acetate, retinol palmitate, dehydroretinol, 9-cis-dehydroretinol, 13-cis-dehydroretinol, 9,13-di-cis-dehydroretinol, retinal, etretinate, retinyl acetate, 9-(4-methoxy-2,3,6-trimethylphenyl)-3,7-dimethyl-2,4,6, 8-nonatetraenoic acid and the like. Especially preferred retinoids include tretinoin and 9-(4-methoxy-2,3,6-trimethylphenyl)-3,7-dimethyl-2,4,6,8-nonatetraenoic acid.

Other contemplated adjuvants include, for example, mannitol, benzoic acid, p-aminobenzoic acid, thioproline, homocysteine, cysteine, acetyl cysteine, dithiothreitol, sodium dithionate, thiosalicyclic acid, mercaptoethanol, ascorbate, thiodipropionic acid, dilauryl thiodipropionate, mercaptoethanolamine, propyl gallate, hydroxyethylruticide, pantothenic acid, and the like. Other suitable adjuvants may be selected from pharmacologically acceptable compounds which have substantial reactivity with hydroxyl radicals, such as those listed in Ross and Ross, "Selected Specific Rates of Reactions of Transients from Water in Aqueous Solution. III. Hydroxyl Radical and Perhydroxyl Radicals and Their Radical Ions", National Standard Reference Data Series, National Bureau of Standards, 59 (1977), and preferably those compounds having a specific rate (k) of at least 5 x 10⁹, and especially at least 10¹⁰.

Another class of adjuvants contemplated as useful herein include antioxidants such as superoxide dismutase (SOD) and compounds having SODase activity, such as, for example, copper aspirinate, indomethocin-copper, and the like. Generally, about 300 units per application is contemplated as an effective amount, but more or less than this may be employed. Also contemplated are basic amino acids, peptides containing basic amino acids (particularly peptides comprised predominantly or entirely of basic amino acids), and metal complexes thereof, such as, for example, glycine, histidine, lysine, arginine, cysteine, methionine, histidyl lysine, glycyl histidine, glycyl hystidyl lysine, lysyl histidyl lysine, etc. Copper, zinc, manganese and iron and other transition metal complexes of these amino acids possess superoxide dismutase activity.

Other compounds which have been found to be helpful as adjuvants in enhancing the hair growth stimulating activity of the free radical-forming compounds include antiandrogens, preferably those which interfer with the binding of androgens such as dihydrotestosterone to receptors in hair follicles. However, antiandrogens which interfere with or inhibit the synthesis of androgenic compounds are also contemplted. Although not fully understood, the preferred antiandrogens appear to function primarily to block dihydrotestosterone receptors rather than to inhibit the reduction of testosterone, and are also known as DHT blockers, but it is also possible that such antiandrogens may function as free radical formers, reducing agents or hydroxyl radical scavengers. Exemplary of such antiandrogens are spironolactone, cyproterone, cyproterone acetate, and the like. Of these, spironolactone is preferred because its effects from topical application are generally more limited to the local site of application.

Effective amounts of the antiandrogen generally range from about 0.01 to about 5 percent by weight of the preparation(s) applied, but more or less than this may be used depending on the particular antiandrogen. The optimum amount is about one percent by weight of the preparation(s) for spironolactone and about 0.1 percent for cyproterone and cyproterone acetate.

In one embodiment of the method, the nitroxide radical forming compound source and the adjuvant are applied as a premixed topical preparation. The nitroxide radical forming compound in this embodiment should be relatively stable in the mixture, and the adjuvant, therefore, must be essentially chemically unreactive with this compound, i.e. to the extent that the mixture contains or remains capable of forming free radicals for a reasonable shelf-life. Preferably, the nitroxide-radical forming compound is at a concentration of e.g. 10-100 mM, and a reducing agent, antioxidant or hydroxyl radical scavenger at a similar concentration. In general, hydroxyl radical scavengers may in some instances be used as solvents at relatively high concentrations. Antioxidants are generally active at trace concentrations, e.g. as low as 0.02 weight percent or lower.

Typical examples may include nicotinamide N-oxide or nicotinic acid N-oxide with ethanol, DMSO, propyl dimethyl phosphine oxide, tretinoin, sodium dithionate, lyslyl-histidyl lysine, and/or copper aspirinate, and the like; and other similar combinations of the above mentioned stable radical formers with one or more of the above mentioned adjuvants. Particularly preferred are combinations of the stable nitroxide radical forming compound with a hydroxyl radical scavenger and a compound with SODase activity. Such combinations of ingredients are generally effective to stimulate hair growth when used as hereinbefore described. In addition, the preparation containing the stable radical former, the hydroxyl radical scavenger and any SODase activity, may also include an antiandrogen.

In some instances, the stable radical former may be a sufficiently effective hair growth stimulant that no adjuvant need be employed, i.e. such stable radical formers may be used without a hydroxyl radical scavenger, or with a relatively weak hydroxyl radical scavenger. Inasmuch as such compounds are previously unknown for topical use as hair growth stimulants, they are within the purview of the present invention. Contemplated examples of these include nicotinic acid N-oxide, nicotinamide N-oxide, tempo, proxyl, doxyl, N-t-butyl-α-phenyl nitrone and the like. However, the activity of the stable radical former is generally enhanced or potentiated by combination with an antioxidant, reducing agent or hydroxyl radical scavenger, and such combinations are therefore preferred.

In another embodiment of the method of the invention, the nitroxide radical forming or generating compound preferably in a pharmaceutical carrier, is admixed witth a reducing agent, also preferably in a pharmaceutical carrier, at or near the time of application to the skin. The nitroxide radical forming compounds reactive with reducing agents to generate free radicals are preferred.

Organic peroxides can also be used to generate free radicals upon reaction with reducing agents. Free radical forming compounds, such as, for example, organic nitrates and inorganic nitrites, react with reducing compounds to form nitric oxide radicals as a reaction product. However, the formation of nitric oxide is generally irreversible since the nitric oxide radical is itself relatively reactive with a half life of about 30 seconds. Thus, it is important in this embodiment of the method to separate the nitric oxide radical-forming compound from the reducing agent until use. Just before application in this embodiment, the nitric oxide radical-forming compound is admixed with the activating agjuvant to initiate formation of the nitric oxide. Then, while the nitric oxide reactants are still being produced by the reaction between the nitrate or nitrite and the reducer, the admixture is applied to the skin at which hair growth stimulation is desired. Alternatively, the nitrate or nitrite may be applied directly to the skin in a first pharmaceutical carrier, and the reducing compound applied in a second pharmaceutical carrier. The order of application is not critical, as long as the nitrate or nitrite reacts with the reducer on or in the skin to release nitric oxide therein, although it may be advantageous to allow a period of time for either the nitric oxide source compound or the reducer to penetrate into the skin so that more nitric oxide is formed in the skin adjacent the hair follicles rather than on the skin surface where the nitric oxide may be released into the ambient air with the result of less effective hair growth stimulation. A period of time of about 15-30 minutes between application of the nitrate or nitrite and the reducer is generally sufficient, although the optimum time may depend on the particular nitrate or nitrite, adjuvant, carrier and skin condition, as these variables may affect skin penetrability. If desired, a compound with SODase activity and/or an antiandrogen may also be present in either preparation, preferably the preparation containing the nitric oxide generating compound, to further enhance the hair growth stimulation.

Another aspect of the invention is the provision of a kit for concurrently measuring and applying a unit dosage of the nitroxide-radical forming compound and an adjuvant. The kit includes a unit dose of the nitroxide-radical forming compound, a unit dose of the adjuvant, means for maintaining the unit doses in an unmixed or uncombined state, and means for dispensing the unit doses. The nitroxide radical forming compound and the adjuvant are generally in association with respective first and second topical pharmaceutical carriers, which may be the same or different carriers. The nitroxide radical forming compound may be, for example, an organic nitrate or an inorganic nitrite, and the adjuvant may be a reducing agent reactive with the nitroxide radical forming compound to form nitric oxide. Organic peroxides may also be used to generate the free radical. If desired, a compound with SODase activity and/or an antiandrogen are included in either unit dose, preferably in the unit dosage of the nitrate or nitrite. The kit optionally further includes means for mixing the nitrate or nitrite unit dose with the unit dosage of the reducing agent.

With reference to specific embodiments of the present kit, one version includes a conventional packaging container such as a box or carton containing a plurality of individually sealed packets of 0.5-1 ml of 0.01-5 weight percent free radical source and any SODase and/or antiandrogen in a suitable carrier, and a like plurality of individually sealed packets of 0.5-1 ml of 10-100 mm reducing agent in a suitable carrier. The packets are foil, plastic, coated paper or the like and are manufactured, filled and sealed as is conventional in the pharmaceutical and cosmetic arts. Preferably, the packets are marked with visual indicia to indicate the contents thereof, i.e., either a "step 1" or a "step 2" packet. Conveniently, each box or carton contains a supply of packets for a series of applications for one or two weeks or a month, for example. In use, a packet of each of the nitroxide radical forming compound and reducing agent are broken or torn open, dispensed from the packet by pouring or squeezing as appropriate, mixed in the palms of the hands, and the mixture is then applied to the scalp or other area of treatment. If desired, a small cup may be included in the kit for mixing the unit dose of the free radical source with that of the reducing agent. Alternatively, the unit dose of nitroxide radical forming compound is applied first to the scalp, followed by the unit dose of the reducing agent, or vice versa. In another version of this kit, the packets comprise three layers of foil or other membraneous material, one interposed between the other two to form two chambers on either side thereof. The nitroxide radical forming compound unit dosage, its respective carrier and any SODase activity and/or antiandrogen are placed in one chamber, and the reducing agent and its respective carrier are placed in the other chamber. When the packet is broken or opened, both chambers are simultaneously dispensed and mixed prior to application, such as, for example, by squeezing the packet to extrude the unit dosages and empty the chamber contents into the palm of the hand.

In an alternative kit embodiment, dual reservoirs for the two preparations are commonly dischargable so that proportionate amounts of each may be dispensed, preferably through a common discharge point. For example, a syringe with a chamber divided into two reservoirs can be employed with a split plunger for discharging a unit dose of each preparation may be employed, or a hand pump with split suction tubes extending into each of the reservoirs.

The invention also includes a topical preparation for stimulating hair growth from skin upon repeated application. The nitroxide radical forming compound may be at a concentration of 0.1 to 20 percent by weight, preferably 0.5 to 3 percent by weight, e.g. 10-100 mM, but more or less than this can be used in some instances for suitable results, depending on the particular compound.

In a preferred embodiment, the topical preparation also includes adjuvant. The adjuvant may be a reducing agent, a hydroxyl radical scavenger, an antioxidant, or a combination thereof, and may also include an antiandrogen, all as described hereinabove. The reducing agent and hydroxyl radical scavenger may be present in the preparation at a concentration similar to that described above for the free radical source, i.e. generally at 0.1-20 weight percent, preferably 1-3 weight percent, although higher concentrations could also be used, while antioxidants such as those with SODase activity are generally present at much lower concentrations, e.g. 0.01-10 mM, or a sufficient amount to provide about 300 units of SODase activity.

In one particular embodiment of the preparation, minoxidil or a suitable substitute therefor is preferably present in the composition in an amount of from about 0.1 to about 20 percent by weight of the composition, more preferably from about 0.5 to about 3 percent by weight, and most preferably about 2 percent by weight. For convenience, reference is made in this embodiment to minoxidil, but it is to be understood that the suitable Suitable hydroxyl radical scavengers for use as an adjuvant in the present invention include DMSO or a substantially equivalent hydroxyl radical scavenger in a synergistically effective amount with the nitroxide radical forming compound. Hydroxyl radical scavengers are, for example, sulfoxides, purines, pyrimidines, thiols, alcohols, halide ions, aromatic hydrocarbons and the like. Hydroxyl radical scavengers suitable in the composition of the present embodiment are those pharmaceutically acceptable hydroxyl radical scavengers which have a hydroxyl radical scavenger effectiveness substantially equivalent to or better than DMSO. Preferred hydroxyl radical scavengers are the alkyl methyl sulfoxides in which the alkyl substituent has from one to about 14 carbon atoms and the β-hydroxyalkyl methyl sulfoxides in which the hydroxyalkyl substituent has from two to about 14 carbon atoms, with dimethyl sulfoxide being particularly preferred. Specific representative examples of such sulfoxides include, in addition to DMSO, hexyl methyl sulfoxide, decyl methyl sulfoxide, dodecyl methyl sulfoxide, tetradecyl methyl sulfoxide, β-hydroxydecyl methyl sulfoxide, β-hydroxydodecyl methyl sulfoxide, β-hydroxytetradecyl methyl sulfoxide, and the like. For convenience, reference is made hereinbelow to DMSO, but it is to be understood that other suitable hydroxyl radical scavengers may be present, partially or entirely, in lieu of DMSO.

For the sulfoxides such as DMSO, the amount used is generally from about 5 to about 25 percent by weight of the composition, preferably about 15-20 percent by weight. Depending on the particular carrier, the amount of DMSO present may be adjusted to avoid phase separation. It has also been found that the hair growth stimulation effected in this embodiment is further improved when an antiandrogen as described above is present.

The mechanism for the hair growth stimulation achieved with stable free radicals and antioxidants, hydroxyl radical scavengers and reducing agents is not fully understood, and the invention is not to be constrained or limited by theory. It is belived that nitroxide radicals are mechanistically involved in stimulating follicle cells to grow hair. The reducing agents are believed to enhance the activity of the free radicals by stimulating their formation, and also by functioning as hydroxyl radical scavengers. The hydroxyl radical scavengers are belived to protect the nitroxide radicals in vivo by reducing the concentration of hydroxyl radicals, thereby inhibiting the inactivation of the nitroxide free radicals by the hydroxyl radicals with which they are extremely reactive. Antioxidants such as SOD similarly protect the stable radicals since they cause the dismutation of superoxide which is known to be involved in the formation of hydroxyl radicals. The antiandrogens could be acting to inhibit the adverse effects of DHT on hair growth, but could also be functioning as hydroxyl radical scavengers. Inasmuch as nitric oxide is known to mimic the vasodilating activity of organic nitrates by stimulating the enzyme guanylate cyclase as reported, for example, in Feelisch et al, European Journal of Pharmacology, vol. 139, pp. 19-30 (1987), it is contemplated that other compounds such as cyclic GMP are also potent hair growth stimulants, and that repeated topical application of effective amounts of cyclic GMP in association with a pharmaceutical carrier can stimulate hair growth at the site of application.

The preparation and use of the composition is illustrated by way of the following examples.

### Example 1

A lotion is prepared by combining the following proportion of ingredients:

**TABLE I**

| Ingredient | Proportion |
|---|---|
| Nicotinamide N-oxide | 1 g |
| Ascorbyl palmitate | 0.1 g |
| Spironolactone | 0.5 g |
| Decylmethyl sulfoxide | 5 g |
| Propylene glycol | 30 ml |
| Ethanol | 20 ml |
| Distilled water | 45 ml |

The preparation is applied at a rate of about 0.5-1 ml one to three times per day to the affected area until hair growth is stimulated in about three months.

### Example 2

A composition is prepared as in Example 1 using the following proportions of ingredients:

**TABLE II**

| Ingredient | Proportion |
|---|---|
| Nicotinic acid N-oxide | 1 g |
| 2-Pyrrolidone | 5 g |
| Dermovan emulsion * | 75 g |
| Distilled water | 20 g |
| Notes for Table II 1. Obtained from Owen Laboratories; Dermovan* emulsion contains water, glycerol stearate, glycerin, mineral oil, synthetic-spermaceti, cetyl alcohol, butylparaben, propylparaben and methylparaben. | |

The distilled water was added to the nicotinic acid-N-oxide in a suitable container. The 2-pyrrilidone was then added and the mixture was thoroughly mixed and allowed to stand overnight. Then, with constant stirring the Dermovan* emulsion was added slowly. The mixture was allowed to stand at least 24 hours with occasional stirring.

### Example 3

Aqueous sodium nitrite at 0.6 weight percent is combined with 2 weight percent cysteine and 0.5-1.0 ml of the mixture is immuliately applied to the affected area. The procecdure is repeated 1-3 times per day, and stimulated hair growth is observed at about 3 months.

### Example 4

A lotion is made by homogenizing the following ingredients:

**TABLE III**

| Ingredient | Proportion |
|---|---|
| N-t-butyl- -phenyl nitrone | 0.1 g |
| Spironolactone | 0.5 g |
| BHT | 0.01 g |
| DMSO | 20 ml |
| Water | 20 ml |
| Dermovan* emulsion | 60 g |

The composition is used as in Example 1.

### Example 5

Example 4 is repeated, but using the following proportions of ingredients:

**TABLE IV**

| Ingredient | Proportion |
|---|---|
| Sodium 3,5-dibromo-4-nitrosobenzene sulfonate | 0.1 g |
| Spironolactone | 0.5 g |
| Retinoic acid | 0.05 g |
| Water | 20 ml |
| BHT | 0.05 g |
| Propylene glycol | 30 g |
| Ethanol | 50 g |

## Claims

1. A method of stimulating hair growth by cosmetic treatment, comprising:
topically applying to skin a pharmacologically acceptable nitroxide radical-forming compound selected from nitroxide spin labels and spin traps and N-oxides of niacis; and
concurrently therewith, topically applying to the skin an adjuvant selected from reducing agents, antioxidants and hydroxyl radical scavengers.

2. A method according to claim 1, wherein applications are repeated over a sufficient period of time to stimulate hair growth.

3. A method of stimulating hair growth by cosmetic treatment, comprising:
mixing, in a pharmaceutical carrier (i) a pharmacologically acceptable stable nitroxide radical-forming compound selected from nitroxide spin labels and spin traps and N-oxides of niacis with (ii) an amount of pharmacologically acceptable reducing agent effective to induce nitroxide radical formation from the stable compound;
applying the nitroxide-forming mixture topically to skin; and
repeating application over a period of time sufficient to stimulate hair growth from the skin.

4. A method according to claim 3, wherein the radical-forming compound and the reducing agent are separated prior to mixing, and the mixture is applied to skin within about one hour following mixing.

5. A method according to claim 3 or 4, wherein the nitroxide radical-forming compound is trinitroglycerin, amyl nitrate, erithrithymyl tetranitrate, isosorbide dinitrate, isosorbide-2-nitrate, isosorbide-5-nitrate, isomannide dinitrate, isomannide-2-nitrate, isoidide dinitrate, or isoidide-2-nitrate.

6. A method according to claim 3 or 4, wherein the nitroxide radical-forming compound is an alkali metal nitrite or transition metal nitrite.

7. A method according to claim 6, wherein the nitroxide radical-forming compound is sodium nitrite.

8. A method according to any of claims 3 to 7, wherein the nitroxide radical-forming compound is present in the mixture at a concentration of about 10 to 100 mM.

9. A method according to any of claims 3 to 8, wherein the amount of reducing agent is at least an equivalent of the radical-forming compound.

10. A kit for preparing a unit dosage of a hair growth stimulating preparation, comprising:
a unit dosage amount of a pharmacologically acceptable nitroxide radical-forming compound selected from organic nitrates and inorganic nitrites in a pharmaceutical carrier;
a unit dosage amount of a pharmacologically acceptable reducing agent in a pharmaceutical carrier, the reducing agent being reactive with the radical-forming compound to form nitroxide radicals;
means for separating the unit dosage amounts from each other prior to dispensing; and
means for dispensing the unit dosage amounts.

11. A kit according to claim 10, wherein the unit dosage amounts are in a total volume of about 0.5 to 1.0 ml.

12. A composition for topical stimulation of hair growth, comprising:
a pharmacologically acceptable stable free radical source compound selected from nitroxide spin labels and N-oxides of niacins and pharmacologically acceptable adjuvant selected from reducing agents, antioxidants and hydroxyl radical scavengers, in association with a topical pharmaceutical carrier.

13. The composition of claim 12, wherein said source compound is present at a concentration of from 0.1 to 20 weight percent and said adjuvant is present at a concentration of from 0.1 to 20 weight percent.

14. The composition of claim 12 or 13, wherein said adjuvant is a hydroxyl radical scavenger having a specific reaction rate constant with hydroxyl radical of at least 5x10⁹dm³/mol-sec.

15. The composition of any of claims 12 to 14, wherein said adjuvant is a hydroxyl radical scavenger having a specific reaction rate constant with hydroxyl radical greater than that of nitric oxide.

16. The composition of any of claims 12 to 15, wherein said adjuvant is selected from dialkyl sulfoxides, tertiary phosphine oxides, retinoids, basic amino acids and basic peptides.

17. The composition of claim 12, wherein said adjuvant comprises dimethyl sulfoxide present at from about 5 to about 25 percent by weight of the composition.

18. The composition of claim 12, wherein said adjuvant comprises dimethyl sulfoxide present at from about 15 to about 20 percent by weight of the composition.

19. The composition of any of claims 12 to 15, wherein said adjuvant is selected from carotene, tretinoin, isotretinoin, 9-cis-dehydroretinol, retinal, etretinate, retinal acetate and 9-(4-methoxy-2,3,6-trimethyl-phenyl)-3,7-dimethyl-2,4,6, 8-nonatetraenoic acid.

20. The composition of any of claims 12 to 15, wherein said adjuvant is selected from mannitol, benzoic acid, p-aminobenzoic acid, thioproline, homocystein, cysteine, acetyl cysteine, dithiothreitol, sodium dithionate, thiosalicylic acid, mercapotethanolamine, propyl gallate, hydroxyethylruticide and pantothenic acid..

21. The composition of any of claims 12 to 15, wherein said adjuvant is selected from the group consisting of basic amino acids, peptides comprised predominantly thereof, and metal complexes of said amino acids and peptides.

22. The composition of claim 21, wherein said basic amino acids are selected from glycine, histidine, lysine, arginine, cysteine, and methionine.

23. The composition of any of claims 12 to 22, further comprising from 0.01 to 5 weight percent of an antiandrogen in association with said carrier.

24. The composition of claim 23, wherein said antiandrogen is spironolactone.

25. The composition of any of claims 12 to 24, wherein said adjuvant includes a compound having SODase activity at a concentration of 0.01-10mM.

26. The composition of claim 25, wherein said SODase compound is selected from superoxide dismutase, copper aspirinate and indomethicin-copper.

27. The composition of any of claims 12 to 26, wherein said free radical source compound is selected from nicotinic acid N-oxide and nicotinamide N-oxide.

28. The composition of claims 12 to 26, wherein said free radical source compound is selected from 4,4-dimethyl-3-oxazolinyloxy ("doxyl"), 3-doxyl-5α -cholestane, 3-doxyl-17β-hydroxy-5α-androstane, 5-doxylstearic acid, 7-doxylstearic acid, 12-doxylstearic acid, 16-doxylstearic acid, 5-doxylstearic acid methyl ester, 7-doxylstearic acid methyl ester, 12-doxylstearic acid methyl ester, and 16-doxylstearic acid methyl ester.

29. The composition of any of claims 12 to 26, wherein said free radical source compound is selected from 2,2,5,5-tetramethyl-1-pyrrolidinyloxyl ("proxyl"), 3-(aminomethyl)-proxyl, 3-(2-[2-bromoacetamido]acetamido)-proxyl, 3-(2-[2-2-bromoacetamido)-ethoxyethyl]-carbamoyl)-proxyl, 3-(2-bromoacetamido]-methyl)-proxyl, 3-(3-[2-bromoacetamido]-proxyl, 3-(2-bromoacetamido]- propylcarbamoyl-proxyl, 3-(2-bromoacetamido)-proxyl, 3-carbamoyl-proxyl, 3-carboxy-proxyl, 3-cyano-proxyl, 3-(5-[dimethylamino] -1-naphthalene-sulfonamido)-proxyl, 3-(5-[dimethylamino]-1-naphthalene-sulfonamido)-proxyl, 3-(5-fluoro-2,4-dinitroanilino)-proxyl, 3-(2-[2-iodoacetamido]acetamido)-proxyl, 3-(2-[2-(-iodoacetamido)-ethoxyethyl]-carbamoyl)-proxyl, 3-(2-iodoacetamidomethyl)-proxyl, 3-(3-[2-iodoacetamido]-ethoxyethyl]-carbamoyl)-proxyl, 3-(2-iodoacetamidomethyl)-proxyl, 3-(3-[2-iodoacetamido]-propylcarbamoyl)-proxyl, 3-(2-iodoacetamido)-proxyl, 3-(2-[2-isothiocyanatoethoxy]-ethylcarbamoyl]-proxyl, 3-(2-isothiocyanatoethylcarbamoyl)-proxyl, 3-(isothiocyanatomethyl)-proxyl, 3-(2-[2-maleimidoethoxy]-ethylcarbamoyl)-proxyl, 3-(2-maleimidoethyl-carbamoyl)-3-(3-maleimidopropylcarbamoyl)-proxyl, 3-maleimidoproxyl, 3-(4-nitrophenoxy carbonyl)-proxyl, and N,N'-bis(3-proxyl carbonyl)-1,2- ethanediamine.

30. The composition of any of claims 12 to 26, wherein asad free radical source compound is selected from 2,2,6,6-tetramethyl-1-piperidinyloxyl ("tempo"), 4-amino-tempo, 4-(2-bromoacetamido)-tempo, 4-[N-formyl-N-(3-hydroxypropyl)amino]-tempo, 4-[N,N-bis-(2-hydroxyethyl)amino-tempo, 4-(ethoxyfluorophosphinyloxy)-tempo, 4-hydroxy-tempo, 4-(2-iodoacetamido)-tempo, 4-isothiocyanato-tempo, 4-maleimido-tempo, 4-(4-nitrobenzoyloxy)-tempo, 4-oxo-tempo, 4-phosphonooxy-tempo, N,N'bis(4-tempo)-oxamide, 1,2-ethanediaminietetraacetic acid, 2',4'-bis[(CN-4-tempo)amide], N,N'-bis(4-tempo)-1,2-ethanediamine, and N,N'-bis(4-tempo)decanediamide.

31. The composition of any of claims 12 to 26, wherein said free radical source compound is selected from 2-(acetoxymercuri)-4,4,4-tetramethyl-2-imidazolin-1-yloxy-3-oxide, 3-carbamoyl-2,5-dihydro-2,2,5,5-tetramethyl-1H-pyrrol-1-yloxy, and 3,([ethoxycarbonyl]-oxycarbonyl)-2,5-dihydro-2,2,5,5-tetramethyl-1H-pyrrol-1-yloxy.

32. The composition of any of claims 12 to 26, wherein said free radical source compound is selected from melanin; N-t-butyl-α-phenyl-nitrone, 3,5-dibromo-4-nitroso-benzenesulfonic acid, 5,5-dimetnyl-1-pyrroline N-oxide, 2-methyl-2-nitroso-propane, nitrosobenzene, nitrosodisulfonic acid, α-(4-pyridyl-1-oxide)-N-t-butylnitrone, 3,3,5,5-tetramethyl-pyrroline N-oxide, and 2,4,6-tri-t-butylnitrosobenzene.

33. The composition of any of claims 12 to 32, wherein the adjuvant is selected from acetyl cystein and suitable pharmacologically acceptable compounds which have substantial reactivity with hydroxyl radicals, such as those listed in Ross and Ross, "Selected Specific Rates of Reactions of Transients from Water in Aqueous Solution. III. Hydroxyl Radical and Perhydroxyl Radicals and Their Radical Ions", National Standard Reference Data Series, National Bureau of Standards, 59 (1977),

34. A method for stimulating hair growth for cosmetic treatment, comprising repeatedly topically applying to the skin a composition as claimed in claim 33 at a concentration and over a period of time sufficient to stimulate hair growth from the skin.

## Patentansprüche

1. Verfahren zur Stimulierung des Haarwuchses durch kosmetische Behandlung, das folgende Stufen umfaßt:
Lokale Aufbringung einer pharmakologisch verträglichen Nitroxidradikale bildenden Verbindung, ausgewählt unter Nitroxid-Spinsonden und -Spinfallen und N-Oxiden von Niacinen, auf die Haut und
gleichzeitige lokale Aufbringung eines Zusatzes, ausgewählt unter Reduktionsmitteln, Antioxydantien und Hydroxylradikalfängern, auf die Haut.

2. Verfahren nach Anspruch 1, wobei die Aufbringungen während einer für die Stimulierung des Haarwuchses ausreichenden Zeitdauer wiederholt werden.

3. Verfahren zur Stimulierung des Haarwuchses durch kosmetische Behandlung, das folgende Stufen umfaßt:
Mischen von (i) einer pharmakologisch verträglichen beständigen Nitroxidradikale bildenden Verbindung, ausgewählt unter Nitroxid-Spinsonden und -Spinfallen und N-Oxiden von Niacinen, mit (ii) einer Menge eines pharmakologisch verträglichen, zur Induktion der Nitroxidradikalbildung aus der beständigen Verbindung geeigneten Reduktionsmittels,
lokale Aufbringung des Nitroxid bildenden Gemisches auf die Haut und
Wiederholung der Aufbringung während einer für die Stimulierung des von der Haut ausgehenden Haarwuchses ausreichenden Zeitdauer.

4. Verfahren nach Anspruch 3, wobei die radikalbildende Verbindung und das Reduktionsmittel vor dem Mischen getrennt werden und das Gemisch innerhalb ca. einer Stunde nach dem Mischen auf die Haut aufgebracht wird.

5. Verfahren nach Anspruch 3 oder 4, wobei die Nitroxidradikal bildende Verbindung Trinitroglycerin, Amylnitrat, Erithrithymyltetranitrat, Isosorbiddinitrat, Isosorbid-2-nitrat, Isosorbid-5-nitrat, Isomanniddinitrat, Isomannid-2-nitrat, Isoididdinitrat oder Isoidid-2-nitrat ist.

6. Verfahren nach Anspruch 3 oder 4, wobei die Nitroxidradikal bildende Verbindung ein Alkalimetallnitrit oder Übergangsmetallnitrit ist.

7. Verfahren nach Anspruch 6, wobei die Nitroxidradikal bildende Verbindung Natriumnitrit ist.

8. Verfahren nach einem der Ansprüche 3 bis 7, wobei die Nitroxidradikal bildende Verbindung im Gemisch in einer Konzentration von ca. 10 bis 100 mM vorliegt.

9. Verfahren nach einem der Ansprüche 3 bis 8, wobei die Menge an Reduktionsmittel wenigstens ein Äquivalent der radikalbildenden Verbindung ist.

10. Ausrüstung für die Herstellung einer Dosiseinheit eines den Haarwuchs stimulierenden Präparats, das folgende Komponenten umfaßt:
eine Dosiseinheitsmenge einer pharmakologisch verträglichen Nitroxidradikal bildenden Verbindung, ausgewählt unter organischen Nitraten und anorganischen Nitriten, in einem pharmazeutischen Träger,
eine Dosiseinheitsmenge eines pharmakologisch verträglichen Reduktionsmittels in einem pharmazeutischen Träger, wobei das Reduktionsmittel mit der radikalbildenden Verbindung unter Bildung von Nitroxidradikalen zu reagieren vermag,
Mittel zur Trennung der Dosiseinheitsmengen voneinander vor der Verteilung und
Mittel zur Verteilung der Dosiseinheitsmengen.

11. Ausrüstung nach Anspruch 10, wobei die Dosiseinheitsmengen in einem Gesamtvolumen von ca. 0,5 bis 1,0 ml vorliegen.

12. Gemisch zur lokalen Stimulierung des Haarwuchses, das folgende Komponenten umfaßt:
Eine pharmakologisch verträgliche beständige Verbindung als Radikalquelle, ausgewählt unter Nitroxid-Spinsonden und N-Oxiden von Niacinen, und einen pharmakologisch verträglichen Zusatz, ausgewählt unter Reduktionsmitteln, Antioxydantien und Hydroxylradikalfängern, in Verbindung mit einem lokalen pharmazeutischen Träger.

13. Gemisch nach Anspruch 12, wobei die als Quelle verwendete Verbindung in einer Konzentration von 0,1 bis 20 Gew.-% und der Zusatz in einer Konzentration von 0,1 bis 20 Gew.-% vorliegen.

14. Gemisch nach Anspruch 12 oder 13, wobei der Zusatz ein Hydroxylradikalfänger mit einer spezifischen Reaktionskonstante mit dem Hydroxylradikal von wenigstens 5 x 10⁹ dm³/Mol-sec ist.

15. Gemisch nach einem der Ansprüche 12 bis 14, wobei der Zusatz ein Hydroxylradikalfänger mit einer spezifischen Reaktionskonstante mit dem Hydroxylradikal, die über der von Stickstoffoxid liegt, ist.

16. Gemisch nach einem der Ansprüche 12 bis 15, wobei der Zusatz ausgewählt wird aus Dialkylsulfoxiden, tertiären Phosphinoxiden, Retinoiden, basischen Aminosäuren und basischen Peptiden.

17. Gemisch nach Anspruch 12, wobei der Zusatz Dimethylsulfoxid darstellt, das in einer Menge von ca. 5 bis ca. 25 Gew.-% des Gemisches enthalten ist.

18. Gemisch nach Anspruch 12, wobei der Zusatz Dimethylsulfoxid darstellt, das in einer Menge von ca. 15 bis ca. 20 Gew.-% des Gemisches enthalten ist.

19. Gemisch nach einem der Ansprüche 12 bis 15, wobei der Zusatz ausgewählt wird unter Carotin, Tretinoin, Isotretinoin, 9-Cis-dehydroretinol, Retinal, Etretinat, Retinalacetat und 9-(4-Methoxy-2,3,6-trimethyl-phenyl)-3,7-dimethyl-2,4,6,8-nonatetraensäure.

20. Gemisch nach einem der Ansprüche 12 bis 15, wobei der Zusatz ausgewählt wird unter Mannit, Benzoesäure, p-Aminobenzoesäure, Thioprolin, Homocystein, Cystein, Acetylcystein, Dithiothreitol, Natriumdithionat, Thiosalicylsäure, Mercaptoethanolamin, Propylgallat, Hydroxyethylruticid und Pantothensäure.

21. Gemisch nach einem der Ansprüche 12 bis 15, wobei der Zusatz ausgewählt wird aus der Gruppe, bestehend aus basischen Aminosäuren, vorwiegend diese enthaltenden Peptiden und Metallkomplexen dieser Aminosäuren und Peptide.

22. Gemisch nach Anspruch 21, wobei die basischen Aminosäuren ausgewählt werden unter Glycin, Histidin, Lysin, Arginin, Cystein und Methionin.

23. Gemisch nach einem der Ansprüche 12 bis 22, das außerdem noch 0,01 bis 5 Gew.-% eines Antiandrogens in Verbindung mit dem Träger enthält.

24. Gemisch nach Anspruch 23, wobei das Antiandrogen Spironolacton ist.

25. Gemisch nach einem der Ansprüche 12 bis 24, wobei der Zusatz eine Verbindung mit einer SODase-Aktivität bei einer Konzentration von 0,01 bis 10 mM enthält.

26. Gemisch nach Anspruch 25, wobei die SODaseverbindung ausgewählt ist aus Superoxiddismutase, Kupferaspirinat und Indomethicin-Kupfer.

27. Gemisch nach einem der Ansprüche 12 bis 26, wobei die freie Radikale bildende Verbindung ausgewählt ist unter Nikotinsäure-N-oxid und Nikotinamid-N-oxid.

28. Gemisch nach Anspruch 12 bis 26, wobei die freie Radikale bildende Verbindung ausgewählt ist unter 4,4-Dimethyl-3-oxazolinyloxy ("Doxyl"), 3-Doxyl-5α-cholestan, 3-Doxyl-17β-hydroxy-5α-androstan, 5-Doxyl-, 7-Doxyl-, 12-Doxyl-, 16-Doxylstearinsäure, 5-Doxyl-, 7-Doxyl-, 12-Doxyl- und 16-Doxylstearinsäuremethylester.

29. Gemisch nach einem der Ansprüche 12 bis 26, wobei die freie Radikale bildende Verbindung ausgewählt ist unter 2,2,5,5-Tetramethyl-1-pyrrolidinyloxyl ("Proxyl"), 3-(Aminomethyl)-proxyl, 3-(2-[2-Bromacetamido]acetamido)-proxyl, 3-(2-[2-2-Bromacetamido)-ethoxyethyl]-carbamoyl)-proxyl, 3-(2-Bromacetamido]-methyl 9-proxyl, 3-(3-[2-Bromacetamido]-proxyl, 3-(2-Bromacetamido]-propylcarbamoyl-proxyl, 3-(2-Bromacetamido)-proxyl, 3-Carbamoyl-proxyl, 3-Carboxy-proxyl, 3-Cyanoproxyl, 3-(5-[Dimethylamino]-1-naphthalin-sulfonamido)-proxyl, 3-(5-(Dimethylamino]-1-naphthalin-sulfonamido)-proxyl, 3-(5-Fluor-2,4-dinitroanilino)-proxyl, 3-(2-[2-Jodacetamido]acetamido)-proxyl, 3-(2-[2-(-Jodacetamido)-ethoxyethyl]-carbamoyl)-proxyl, 3-(2-Jodacetamidomethyl)-proxyl, 3-(3-[2-Jodacetamido]-ethoxyethyl]-carbamoyl)-proxyl, 3-(2-Jodacetamidomethyl)-proxyl, 3-(3-[2-Jodacetamido]-propylcarbamoyl)-proxyl, 3-(2-Jodacetamido)-proxyl, 3-(2-[2-Isothiocyanatethoxy]-ethylcarbamoyl]-proxyl, 3-(2-Isothiocyanatethylcarbamoyl)-proxyl, 3-(Isothiocyanatmethyl)-proxyl, 3-(2-[2-Maleimidethoxy]-ethylcarbamoyl)-proxyl, 3-(2-Maleimidethyl-carbamoyl)-3-(3-maleimidpropylcarbamoyl)-proxyl, 3-Maleimidproxyl, 3-(4-Nitrophenoxycarbonyl)-proxyl und N,N'-Bis-(3-proxylcarbonyl)-1,2-ethandiamin.

30. Gemisch nach einem der Ansprüche 12 bis 26, wobei die freie Radikale bildende Verbindung ausgewählt ist unter 2,2,-6,6-Tetramethyl-1-piperidinyloxyl ("Tempo"), 4-Amino-tempo, 4-(2-Bromacetamido)-tempo, 4-(N-Formyl-N-(3-hydroxypropyl)amino]-tempo, 4-(N,N-Bis-(2-hydroxyethyl)amino-tempo, 4-(Ethoxyfluorphosphinyloxy)-tempo, 4-Hydroxy-tempo, 4-(2-Jodacetamido)-tempo, 4-Isothiocyanat-tempo, 4-Maleimid-tempo, 4-(4-Nitrobenzoyloxy)-tempo, 4-Oxo-tempo, 4-Phosphonoxy-tempo, N,N'-Bis(4-tempo)-oxamid, 1,2-Ethandiamintetraessigsäure, 2',4'-Bis[(CN-4-tempo)amid], N,N'-Bis(4-tempo)-1,2-ethandiamin und N,N'-Bis-(4-tempo)decandiamid.

31. Gemisch nach einem der Ansprüche 12 bis 26, wobei die freie Radikale bildende Verbindung ausgewählt ist unter 2-(Acetoxymercuri)-4,4,4-tetramethyl-2-imidazolin-1-yloxy-3-oxid, 3-Carbamoyl-2,5-dihydro-2,2,5,5-tetramethyl-1H-pyrrol-1-yloxy und 3-([Ethoxycarbonyl]-oxycarbonyl)-2,5-dihydro-2,2,5,5-tetramethyl-1H-pyrrol-1-yloxy.

32. Gemisch nach einem der Ansprüche 12 bis 26, wobei die freie Radikale bildende Verbindung ausgewählt ist unter Melanin, N-t-Butyl-α-phenyl-nitron, 3,5-Dibrom-4-nitrosobenzolsulfonsäure, 5,5-Dimethyl-1-pyrrolin-N-oxid, 2-Methyl-2-nitroso-propan, Nitrosobenzol, Nitrosodisulfonsäure, α-(4-Pyridyl-1-oxid)-N-t-butylnitron, 3,3,5,5-Tetramethyl-pyrrolin-N-oxid und 2,4,6-Tri-t-butylnitrosobenzol.

33. Gemisch nach einem der Ansprüche 12 bis 32, wobei der Zusatz ausgewählt ist unter Acetylcystein und geeigneten pharmakologisch verträglichen Verbindungen, die gut mit Hydroxylradikalen zu reagieren vermögen, wie beispielsweise die in Ross end Ross "Selected Specific Rates of Reactions of Transients from Water in Aqueous Solution. III. Hydroxyl Radical and Perhydroxyl Radicals and Their Radical Ions", National Standard Reference Data Series, National Bureau of Standards, 59 (1977) aufgeführten Verbindungen.

34. Verfahren zur Stimulierung des Haarwachstums für die kosmetische Behandlung, das die wiederholte lokale Auftragung eines Gemisches nach Anspruch 33 in einer Konzentration und während einer Zeitdauer, die für die Stimulierung des Haarwachstums von der Haut aus ausreichen, auf die Haut umfaßt.

## Revendications

1. Procédé de stimulation de la pousse des cheveux par traitement cosmétique, comprenant :
l'application en topique sur la peau d'un composé formant des radicaux nitroxyle pharmacologiquement acceptable, choisi parmi les marqueurs de spin et les piégeurs de spin de type nitroxyle et les N-oxydes de niacines ; et
simultanément, l'application en topique sur la peau d'un adjuvant choisi parmi les agents réducteurs, les anti-oxydants et les piégeurs de radicaux hydroxyle.

2. Procédé selon la revendication 1, dans lequel les applications sont répétées pendant une durée suffisante pour stimuler la pousse des cheveux.

3. Procédé de stimulation de la pousse des cheveux par un traitement cosmétique, comprenant :
le mélange, dans un véhicule pharmaceutique, (i) d'un composé formant des radicaux nitroxyle pharmacologiquement acceptable, choisi parmi les marqueurs de spin et les piégeurs de spin de type nitroxyle et les N-oxydes de niacines avec (ii) une quantité pharmacologiquement acceptable d'agent réducteur efficace pour provoquer la formation de radicaux nitroxyle à partir du composé stable ;
l'application en topique sur la peau du mélange formant le nitroxyle ; et
l'application répétée pendant une durée suffisante pour stimuler la pousse des cheveux sur la peau.

4. Procédé selon la revendication 3, dans lequel le composé formant des radicaux et l'agent réducteur sont séparés avant d'être mélangés, et le mélange est appliqué sur la peau environ une heure après le mélange.

5. Procédé selon la revendication 3 ou 4, dans lequel le composé formant des radicaux nitroxyle est le trinitroglycérol, le nitrate d'amyle, le tétranitrate d'érythrithymyle, le dinitrate d'isosorbide, l'isosorbide-2-nitrate, l'isosorbide-5-nitrate, le dinitrate d'isomannide, l'isomannide-2-nitrate, le dinitrate d'isoidide ou l'isoidide-2-nitrate.

6. Procédé selon la revendication 3 ou 4, dans lequel le composé formant des radicaux nitroxyle est un nitrite de métal alcalin ou un nitrite d'un métal de transition.

7. Procédé selon la revendication 6, dans lequel le composé formant des radicaux nitroxyle est le nitrite de sodium.

8. Procédé selon l'une quelconque des revendications 3 à 7, dans lequel le composé formant des radicaux nitroxyle est présent dans le mélange à une concentration d'environ 10 à 100 mM.

9. Procédé selon l'une quelconque des revendications 3 à 8, dans lequel la quantité d'agent réducteur est d'au moins un équivalent du composé formant des radicaux.

10. Trousse de préparation d'une dose unitaire d'une préparation stimulant la pousse des cheveux, comprenant :
une quantité posologique unitaire d'un composé formant des radicaux nitroxyle pharmacologiquement acceptable, choisi parmi les nitrates organiques et les nitrites minéraux, dans un véhicule pharmaceutique ;
une quantité posologique unitaire d'un agent réducteur pharmacologiquement acceptable dans un véhicule pharmaceutique, l'agent réducteur étant réactif avec le composé formant des radicaux, pour former des radicaux nitroxyle ;
un dispositif pour séparer les quantités posologiques unitaires les unes des autres avant de les administrer ; et
un dispositif pour administrer les quantités posologiques unitaires.

11. Trousse selon la revendication 10, dans laquelle les quantités posologiques unitaires forment un volume total d'environ 0,5 à 1,0 ml.

12. Composition pour la stimulation locale de la pousse des cheveux comprenant :
un composé source de radicaux libres stables pharmacologiquement acceptable, choisi parmi les marqueurs de spin de type nitroxyle et les N-oxydes de niacines, et un adjuvant pharmacologiquement acceptable, choisi parmi les agents réducteurs, les anti-oxydants et les piégeurs de radicaux hydroxyle, en association avec un véhicule pharmaceutique à usage local.

13. Composition selon la revendication 12, dans laquelle ledit composé source est présent à une concentration de 0,1 à 20% en poids et ledit adjuvant est présent à une concentration de 0,1 à 20 % en poids.

14. Composition selon la revendication 12 ou 13, dans laquelle ledit adjuvant est un piégeur de radicaux hydroxyle possédant une constante de vitesse réactionnelle spécifique avec le radical hydroxyle d'au moins 5 x 10⁹ dm³/mole.s.

15. Composition selon l'une quelconque des revendications 12 à 14, dans laquelle ledit adjuvant est un piégeur de radicaux hydroxyle possédant une constante de vitesse réactionnelle spécifique avec le radical hydroxyle supérieure à celle de l'oxyde nitrique.

16. Composition selon l'une quelconque des revendications 12 à 15, dans laquelle ledit adjuvant est choisi parmi les dialkylsulfoxydes, les oxydes de phosphine tertiaires, les rétinoïdes, les acides aminés basiques et les peptides basiques.

17. Composition selon la revendication 12, dans laquelle ledit adjuvant comprend du diméthylsulfoxyde, présent à raison d'environ 5 à environ 25 % en poids de la composition.

18. Composition selon la revendication 12, dans laquelle ledit adjuvant comprend du diméthylsulfoxyde, présent à raison d'environ 15 à environ 20% en poids de la composition.

19. Composition selon l'une quelconque des revendications 12 à 15, dans laquelle ledit adjuvant est choisi parmi le carotène, la trétinoïne, l'isotrétinoïne, le 9-cis-déshydrorétinol, le rétinal, l'étrétinate, l'acétate de rétinal et l'acide 9-(4-méthoxy-2,3,6-triméthylphényl)-3,7-diméthyl-2,4,6,8-nonatétraénoïque.

20. Composition selon l'une quelconque des revendications 12 à 15, dans laquelle ledit adjuvant est choisi parmi le mannitol, l'acide benzoïque, l'acide p-aminobenzoïque, la thioproline, l'homocystéine, la cystéine, l'acétylcystéine, le dithiothréitol, le dithionate de sodium, l'acide thiosalicylique, la mercaptoéthanolamine, le gallate de propyle, l'hydroxyéthylruticide et l'acide pantothénique.

21. Composition selon l'une quelconque des revendications 12 à 15, dans laquelle ledit adjuvant est choisi dans le groupe constitué par les acides aminés basiques, les peptides composés principalement d'acides aminés basiques et les complexes métalliques desdits acides aminés et peptides.

22. Composition selon la revendication 21, dans laquelle lesdits acides aminés basiques sont choisis parmi la glycine, l'histidine, la lysine, l'arginine, la cystéine et la méthionine.

23. Composition selon l'une quelconque des revendications 12 à 22, comprenant en outre de 0,01 à 5 % en poids d'un anti-androgène en association avec ledit véhicule.

24. Composition selon la revendication 23, dans laquelle ledit anti-androgène est la spironolactone.

25. Composition selon l'une quelconque des revendications 12 à 24, dans laquelle ledit adjuvant comprend un composé ayant une activité SODase, à une concentration de 0,01-10mM.

26. Composition selon la revendication 25, dans laquelle ledit composé SODase est choisi parmi la super-oxyde dismutase, l'aspirinate de cuivre et l'indométhacine-cuivre.

27. Composition selon l'une quelconque des revendications 12 à 26, dans laquelle ledit composé source de radicaux libres est choisi parmi le N-oxyde d'acide nicotinique et le N-oxyde de nicotinamide.

28. Composition selon les revendications 12 à 26, dans laquelle ledit composé source de radicaux libres est choisi parmi le 4,4-diméthyl-3-oxazolinyloxy ("doxyle"), le 3-doxyl-5Ò-cholestane, le 3-doxyl-17β-hydroxy-5Ò-androstane, l'acide 5-doxylstéarique, l'acide 7-doxylstéarique, l'acide 12-doxylstéarique, l'acide 16-doxylstéarique, l'ester méthylique d'acide 5-doxylstéarique, l'ester méthylique d'acide 7-doxylstéarique, l'ester méthylique d'acide 12-doxylstéarique et l'ester méthylique d'acide 16-doxylstéarique.

29. Composition selon l'une quelconque des revendications 12 à 26, dans laquelle ledit composé source de radicaux libres est choisi parmi le 2,2,5,5-tétraméthyl-1-pyrrolidinyloxyle ("proxyle"), le 3-(aminométhyl)proxyle, le 3-(2-[2-bromoacétamido]acétamido)proxyle, le 3-(2-[2-bromoacétamidoéthoxyéthyl]carbamoyl)proxyle, le 3-(2-bromoacétamido-méthyl)proxyle, le 3-(3-[2-bromoacétamido]proxyle, le 3-(2-bromoacétamido)propylcarbamoyl-proxyle, le 3-(2-bromoacétamido)proxyle, le 3-carbamoyl-proxyle, le 3-carboxy-proxyle, le 3-cyano-proxyle, le 3-(5-[diméthylamino]-1-naphtalène-sulfonamido)proxyle, le 3-(5-fluoro-2,4-dinitroanilino)proxyle, le 3-(5-[diméthylamino]-1-naphalène-sulfonamido)-proxyle, le 3-(2-[2-iodoacétamido]acétamido)proxyle, le 3-(2-[2-(iodoacétamido)éthoxyéthyl]carbamoyl)proxyle, le 3-(2-iodoacétamidométhyl)proxyle, le 3-(3-[2-iodoacétamido-éthoxyéthyl]carbamoyl)proxyle, le 3-(2-iodoacétamidométhyl)-proxyle, le 3-(3-[2-iodoacétamido]propylcarbamoyl)proxyle, le 3-(2-iodoacétamido)proxyle, le 3-(2-[2-isothiocyanatoéthoxy]éthylcarbamoyl)proxyle, le 3-(2-isothiocyanatoéthylcarbamoyl)proxyle, le 3-(¹isothiocyanatométhyl)proxyle, le 3-(2-[2-maléimidoéthoxy]éthylcarbamoyl)proxyle, le 3-(2-maléimidoéthylcarbamoyl)-3-(3-maléimidopropylcarbamoyl)proxyle, le 3-maléimidoproxyle, le 3-(4-nitrophénoxycarbonyl)proxyle et la N,N'-bis(3-proxylcarbonyl)-1,2-éthanediamine.

30. Composition selon l'une quelconque des revendications 12 à 26, dans laquelle ledit composé source de radicaux libres est choisi parmi le 2,2,6,6-tétraméthyl-1-pipéridinyloxyle ("tempo"), le 4-amino-tempo, le 4-(2-bromoacétamido)tempo, le 4-[N-formyl-N-(3-hydroxypropyl)amino]tempo, le 4-[N,N-bis-(2-hydroxyéthyl)amino-tempo, le 4-(éthoxyfluorophosphinyloxy)tempo, le 4-hydroxytempo, le 4-(2-iodoacétamido)tempo, le 4-isothiocyanato-tempo, le 4-maléimidotempo, le 4-(4-nitrobenzoyloxy)tempo, le 4-oxo-tempo, le 4-phosphonoxy-tempo, le N,N'-bis-(4-tempo)oxamide, l'acide 1,2-éthanediaminetétraacétique, le 2',4'-bis[(CN-4-tempo)amide], la N,N'-bis-(4-tempo)-1,2-éthanediamine et le N,N'-bis-(4-tempo)décanediamide.

31. Composition selon l'une quelconque des revendications 12 à 26, dans laquelle ledit composé source de radicaux libres est choisi parmi le 2-(acétoxymercuri)-4,4,4,4-tétraméthyl-2-imidazoline-1-yloxy-3-oxyde, le 3-carbamoyl-2,5-dihydro-2,2,5,5-tétraméthyl-1H-pyrrole-1-yloxy et le 3-([éthoxycarbonyl]oxycarbonyl)-2,5-dihydro-2,2,5,5-tétraméthyl-1H-pyrrole-1-yloxy.

32. Composition selon l'une quelconque des revendications 12 à 26, dans laquelle ledit composé source de radicaux libres est choisi parmi la mélanine, la N-t-butyl-Ò-phényl-nitrone, l'acide 3,5-dibromo-4-nitrosobenzènesulfonique, le N-oxyde de 5,5-diméthyl-1-pyrroline, le 2-méthyl-2-nitrosopropane, le nitrosobenzène, l'acide nitrosodisulfonique, l'Ò-(4-pyridyl-1-oxyde)-N-t-butylnitrone, le N-oxyde de 3,3,5,5-tétraméthylpyrroline et le 2,4,6-tri-t-butylnitrosobenzène.

33. Composition selon l'une quelconque des revendications 12 à 32, dans laquelle l'adjuvant est choisi parmi l'acétylcystéine et les composés pharmaceutiquement acceptables qui possèdent une réactivité substantielle avec les radicaux hydroxyle, tels que ceux énumérée dons Ross & Ross, "Selected Specific Rates of Reactions of Transcients from Water in Aqueous Solution, III. Hydroxyl Radical and Perhydroxyl Radicals and Their Radical Ions", National Standard Reference Data Series, National Bureau of Standards, 59 (1977).

34. Procédé de stimulation de la pousse des cheveux par traitement cosmétique, comprenant l'application répétée en topique sur la peau d'une composition selon la revendication 33, à une concentration et pendant une durée suffisantes pour stimuler la pousse des cheveux sur la peau.
